**Europäisches Patentamt**

**European Patent Office**

**Office européen des brevets**

(19)

(11) Veröffentlichungsnummer: **0 444 460 A2**

## (12) EUROPÄISCHE PATENTANMELDUNG

(21) Anmeldenummer: 91101835.6

(22) Anmeldetag: 09.02.91

(51) Int. Cl.5: **C07C 45/51, C07C 47/22**

(30) Priorität: 17.02.90 DE 4005163

(43) Veröffentlichungstag der Anmeldung:
04.09.91 Patentblatt 91/36

(84) Benannte Vertragsstaaten:
**BE CH DE ES FR GB IT LI NL**

(71) Anmelder: **BASF Aktiengesellschaft
Carl-Bosch-Strasse 38
W-6700 Ludwigshafen(DE)**

(72) Erfinder: **Steck, Werner, Dr.
Auerstrasse 4
W-6700 Ludwigshafen(DE)**
Erfinder: **Schwarzmann, Matthias, Dr.
Carl-Bosch-Strasse 54
W-6703 Limburgerhof(DE)**
Erfinder: **Weinacht, Kurt
Knietschstrasse 4
W-6703 Limburgerhof(DE)**
Erfinder: **Merger, Franz, Dr.
Max-Slevogt-Strasse 25
W-6710 Frankenthal(DE)**

(54) Verfahren zur Herstellung von Acrolein.

(57) Verfahren zur Isomerierung von Propargylalkohol zu Acrolein, in dem man Propargylalkohol an Heterogenkatalysatoren, die alkalisch reagierende Metallionen enthalten, in der Gasphase bei Temperaturen von 300 bis 550°C und Drücken von 0,01 bis 50 bar umlagert, sowie die Verwendung von Propargylalkohol als Depotsubstanz für die Freisetzung von Acrolein.

EP 0 444 460 A2

Die vorliegende Erfindung betrifft ein Verfahren zur Herstellung von Acrolein durch heterogen katalysierte Isomerisierung von Propargylalkohol in der Gasphase an Heterogenkatalysatoren, die alkalisch reagierende Metallionen enthalten.

Zur Herstellung von Acrolein sind bereits eine Reihe von Herstellverfahren beschrieben worden. Gemäß Ullmann's Encyclopedia of Industrial Chemistry, Volume A 1, (1985), S. 149 bis 160, kann Acrolein durch Oxidation von Propen oder durch Gasphasenkondensation von Acetaldehyd und Formaldehyd synthetisiert werden. Aufgrund seiner Polymerisationsneigung und Toxikologie ist jedoch eine Vorratshaltung von Acrolein nur beschränkt und nur unter aufwendigen Vorkehrungen und Sicherheitsmaßnahmen möglich.

Es bestand daher die Aufgabe, für den benötigten Bedarf an Acrolein eine Depotsubstanz aus der erst vor Ort und zum gewünschten Zeitpunkt Acrolein freigesetzt und chemischen Umsetzungen zugeführt werden kann, zu finden, die sich leicht zum gewünschten Wertprodukt umsetzen läßt.

Demgemäß wurd ein neues Verfahren zur Isomerierung von Propargylalkohol zu Acrolein gefunden, welches dadurch gekennzeichnet ist, daß man Propargylalkohol an Heterogenkatalysatoren, die alkalisch reagierende Metallionen enthalten, in der Gasphase bei Temperaturen von 300 bis 550 °C und Drücken von 0,01 bis 50 bar umlagert.

Propargylalkohol (Propin-2-ol-1) ist technisch leicht aus Acetylen und Formaldehyd erhältlich.

Damit bietet sich beispielsweise die Möglichkeit in einem vorgeschalteten Reaktor Acrolein aus Propargylalkohol freizusetzen und dieses einer chemischen Reaktion zuzuführen. In anderen Fällen kann Propargylalkohol als verkapptes Acrolein sogar direkt zur Umsetzung in situ, beispielsweise mit Dienen eingesetzt werden.

Die für die erfindungsgemäße Umwandlung in der Gasphase geeigneten Reaktionsbedingungen liegen bei Temperaturen von 300 bis 550 °C und Drücken von 0,01 bis 50 bar, vorzugsweise Atmosphärendruck und beispielsweise einer Belastung WHSV von 0,8 bis 8 $h^{-1}$ (Dimension der WHSV = g Einsatzgemisch/g Katalysator und Stunde). Die Reaktion läßt sich diskontinuierlich oder vorzugsweise kontinuierlich beispielsweise bei Normaldruck in einem Festbett, Fließ- oder Wirbelbett, ggf. mit kontinuierlicher Katalysatorregenerierung, ausführen.

Die Reaktion kann in Gegenwart oder unter gleichzeitigem Durchleiten von Inertgasen, wie $N_2$, $CO_2$ oder Edelgasen durchgeführt werden. Die Durchführung erfolgt bei allen Varianten vorzugsweise kontinuierlich. Solange eine ausreichende Verweilzeit gewährleistet bleibt, kann auch unter vermindertem Druck von 0,01 bis 0,99 bar gearbeitet werden.

Als Katalysatoren für das erfindungsgemäße Verfahren eignen sich Heterogenkatalysatoren, welche beispielsweise Kationen der Alkali-und/oder Erdalkalimetalle enthalten.

Die erfindungsgemäße Isomerisierung beruht vermutlich teilweise auf dem bekannten, basischen Charakter der genannten Kationen in Form ihrer Oxide, Hydroxide oder Salze. So haben sich beispielsweise die Hydroxide NaOH, KOH, CsOH, Ca(OH)₂, Sr(OH)₂, Ba(OH)₂ oder die Oxide MgO, CaO, CaO, SrO und BaO als geeignet erwiesen. Dabei können diese Ionen, Salze, Oxide oder sonstigen Verbindungen teils in reiner Form, z.B. als MgO; teils in Molekularsieben wie Zeolithen und strukturverwandten Phosphaten inkorporiert oder auf Trägermaterialien aufgebracht verwendet werden. Als Trägermaterialien eignen sich beispielsweise $Al_2O_3$ und andere Aluminiumoxide, Aluminiumsilikate, Magnesiumaluminiumsilikate und Tonmineralien, $SiO_2$ und andere Si-haltige Trägermaterialien oder gefällte Metallphosphate von zwei- und dreiwertigen Kationen wie Ca, Sr, Ba oder Al und Bor.

Die besondere Eignung des Katalysators und damit die Bestimmung einer ausreichenden Menge an katalytisch aktiven Ionen oder Verbindungen läßt sich durch einige Maßnahmen charakterisieren. Zunächst kann der pH-Wert einer wässrigen Katalysatorsuspension dadurch bestimmt werden, daß z.B. 1 g des Katalysators in 10 g destilliertem Wasser 10 Minuten lang gekocht wird, danach der Katalysator abfiltriert und dann der pH-Wert des klaren Filtrates nach dem Abkühlen auf 22 °C oder unter Temperaturkompensation am PH-Meter gemessen wird. Dieser pH-Wert sollte mindestens 8 betragen. Häufig liegt der pH gut geeigneter Katalysatoren jedoch bei 9 und höher.

Weiterhin können mit Hilfe einer von R.M. Dessau publizierten basenkatalysierten, intramolekularen Gasphasenzyklisierung von Acetonylaceton, einem 1,4-Diketon, zum 3-Methylcyclopentenon Hinweise auf eine mögliche Eignung des betrachteten Katalysators gefunden werden (Literatur: R.M. Dessau, Recent Research Report 214, p. 457-458 of the International Zeolite Conference, Amsterdam, July 10-14, 1989). Beim Vorliegen saurer oder überwiegend saurer Eigenschaften des Katalysators wird dagegen das Diketon zu 2,5-Methylfuran und nicht wie bei der Basenkatalyse zum 3-Methylpentenon zyklisiert. Amphotere Katalysatoren, die sowohl saure als auch basische Eigenschaften aufweisen, können in der genannten Reaktion beide Heterozyklen in wechselnden Anteilen gleichzeitig erzeugen.

Für das erfindungsgemäße Verfahren geeignete Katalysatoren führen bei einer Test-Temperatur von 350 °C und einer Belastung WHSV von 4/h in der Regel zu einem Anteil von mindestens 70 % 3-Methylcyclopentenon neben 2,5-Dimethylfuran. Es ist also möglich, daß für das erfindungsgemäße Verfahren auch amphotere Kontakte geeignet sind, wenn in diesen die basische Eigenschaften überwiegen.

Eine Möglichkeit der Aufbringung der erfindungsgemässen Metallionen auf Trägermaterialien ist z. B. dadurch gegeben, daß man den Träger z. B. mit einem Hydroxid, einem Nitrat, einem Carbonat, einem Oxid oder Alkoholat der voranbeschriebenen Kationen in wäßriger, alkoholischer oder ammoniakalischer Lösung, z. B. durch Einrotieren am Rotationsverdampfer, imprägniert. In der Regel schließt sich zumindest eine Trocknung, wahlweise auch eine Calcinierung beispielsweise bei 400 bis 700° C an. Dabei können gegebenenfalls die auf dem zuvor getränkten Träger befindlichen Carbonate, Hydroxide, Hydrate oder Nitrate in die als Basen wirksamen Alkali- oder Erdalkalioxide überführt werden. Geeignete Katalysatoren können auch durch Tränken der frisch gefällten Trägermaterialien, wie beispielsweise Phosphate oder Kieselgele, mit Lösungen von Alkali- und/oder Erdalkalimetallen erhalten werden. Desgleichen können die Alkali- oder Erdalkaliionen bereits bei der Fällung von Trägermaterialien zugegen sein.

Als Katalysatoren für das erfindungsgemäße Verfahren können auch zeolithische oder ihnen strukturverwandte Festkörper, d. h. Molekularsiebe, als Katalysatoren eingesetzt werden, welche ihre besonderen katalytischen Eigenschaften durch Ioneneintausch, durch Imprägnierung oder sonstige Modifizierung mit Alkali- oder Erdalkaliionen erhalten.

Entsprechend ihrer Struktur werden Zeolithe in verschiedene Gruppen unterteilt. Je nach Verknüpfung ihrer elementaren Struktureinheiten entstehen unterschiedlich große Hohlräume und Poren. Man unterscheidet daher die Zeolithe u. a. in die Typen A, L, X, Y oder Pentasile. Die chemische Zusammensetzungen und die Strukturen für beispielsweise Mordenit und Faujasit sind im "Atlas of Zeolite Structure Types", W.M. Meier and D.H. Olsen, Butterworths, Edition 1987 angegeben.

Für das erfindungsgemäße Verfahren geeignete Zeolithe gehören beispielsweise dem Pentasiltyp an, welcher als Struktureinheit einen aus TO$_4$-Tetraedern aufgebauten Fünfring aufweist, wobei als T-Kation beispielsweise Si, Al, Ga, B oder Fe verwendet werden können. Die Definition der Pentasil-Zeolithe stammt von G.T. Kokotailo und W.M. Meier, Chem. Soc. Spec. Publ. (1980) 33, Seite 133-139. Pentasilzeolithe sind durch Porengrößen gekennzeichnet, die zwischen denen der Zeolithe vom Typ A und denen vom Typ X oder Y liegen. Die Herstellung dieser Pentasil-Zeolithe wird u.a. in den US-A-3 702 886 (ZSM-5), US-A-3 709 979 (ZSM-11) und US-A-4 061 724 (Silicalite®) beschrieben. Auch gehören hierzu die isotaktischen Pentasil-Zeolithe nach EP-A-34 727 oder Eisensilikatzeolithe nach DE-A-27 55 770.

Liegt der Zeolith aufgrund der Art seiner Herstellung nicht in der katalytisch erwünschten Form vor, sondern z.B. in der sauren H-Form, dann kann diese durch Ionenaustausch oder durch Dotierung bzw. Imprägnierung mit Alkali-Ionen wie Li, Na, K oder Cs oder Erdalkali-Ionen wie Mg, Ca, Sr oder Ba in die gewünschte Form überführt werden. Es wird dabei angenommen, daß auf oder im Katalysator vorhandene Hydroxide wie NaOH, KOH, CsOH bzw. Oxide wie MgO oder CaO oder auch Hydroxide wie Ba(OH)$_2$ für die besonderen Eigenschaften des Katalysators von Bedeutung sind.

Eine geeignete Modifizierung Katalysatoren mit Alkali- oder Erdalkaliionen besteht z. B. darin, daß man den unverformten oder verformten Zeolithen mit Metallsalzen durch einen Ionenaustausch oder durch Imprägnierung dotiert.

Zweckmäßigerweise führt man die Dotierung so durch, daß man z. B. den verformten Zeolithen in einem Steigrohr vorlegt und bei 20 bis 100° C, z.B. eine wäßrige Lösung eines Hydroxids der voranbeschriebenen Kationen überleitet. Ein derartiger Ionenaustausch kann z. B. an der Wasserstoff-, Ammonium-, oder Alkaliform des Zeolithen vorgenommen werden.

Eine weitere Möglichkeit der Metallionen-Aufbringung auf den Zeolithen ist gegeben, indem man das zeolithische Material z. B. mit einem Hydroxid, einem Carbonat, einem Oxid oder Alkoholat der voranbeschriebenen Kationen in wäßriger, alkoholischer oder ammoniakalischer Lösung, z.B. durch Einrotieren am Rotationsverdampfer, imprägniert. Sowohl an einen Ionenaustausch als auch an eine Imprägnierung schließt sich zumindest eine Trocknung, wahlweise auch eine Calcinierung beispielsweise bei 400 bis 700° C an. Dabei können gegebenenfalls auch Nitrate, Carbonate, Hydroxide oder Hydrate in die entsprechenden Alkali-oder Erdalkalioxide überführt werden.

Anstelle der zeolithischen Molekularsiebe können auch die unter hydrothermalen Bedingungen synthetisierbaren Aluminiumphosphate oder Siliciumaluminophosphate eingesetzt werden. Diese kristallinen Festkörper weisen den Zeolithen verwandte, definierte Hohlraum- und Porenstrukturen auf. Die Herstellung, Eigenschaften und die Klassifizierung dieser Festkörper auf der Basis von Struktur und chemischer Zusammensetzung werden ausführlich von R.M. Barrer, Pure and Appl. Chem., Vol 58, No 10, pp. 1317-22, (1986); von E.M. Flanigen et al, Pure and Appl. Chem., Vol 58, No. 10, pp 1351-58, (1986) oder von N.B. Milestone et al, Stud. Surf.Sci. Catal. (1988), 36 (Methane Convers.), pp 553-62 beschrieben.

Acrolein ist ein wichtiges Zwischenprodukt und eignet sich für die Synthese von Methionin, Acrylsäure, Glutaraldehyd, Pyridine und andere wichtige chemische Stoffe. In Ullmann's Encyclopedia of Industrial

3

Chemistry 1985, Vol. A, S. 156 und 157, sind die wichtigsten Einsatzgebiete für Acrolein als Zwischenprodukt zusammengestellt.

Beispiele

Gasphasenreaktion (allgemeine Reaktionsbeschreibung für Tabelle 1)

Die Reaktionen in der Gasphase wurden unter isothermen Bedingungen in einem Rohrreaktor (Wendel, 0,6 cm Innendurchmesser, 90 cm Länge) an einem Katalysatorfestbett durchgeführt. Dabei wurde die Katalysatormenge zwischen 1 und 10 g, entsprechend einer Belastung WHSV zwischen von 0,8 und 8/h variiert. Propargylakohol wurde mittels einer Pumpe in den Reaktor in einer Menge von 10 ml/h eindosiert und spätestens am Katalysator-Festbett verdampft. Als Schutz- und Trägergas wurde ein Stickstoffstrom von 10 Nl/h verwendet. Die Reaktionsprodukte wurden in einer Kühlfalle kondensiert und gaschromatographisch analysiert.

Die Ergebnisse sind in der Tabelle 1 zusammengefaßt.

Die verwendeten Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelgut eingesetzt werden.

Tabelle 1: Isomerisierung von Propargylakohol
(V = Vergleichsbeispiele)

| Beispiel Nr. | Kat. Nr. | Kat. [g] | T [°C] | Std | Umsatz Propargyl-alkohol | Ausbeute Acrolein | Selektivität Acrolein |
|---|---|---|---|---|---|---|---|
| V 1 | 1 | 4 | 350 | 4 | 0,4 | 0,4 | 98,1 |
|  |  |  | 450 | 6 | 3,4 | 2,8 | 80,9 |
| 2 | 2 | 2 | 400 | 2 | 25,1 | 24,5 | 97,4 |
| 3 | 3 | 2 | 350 | 2 | 24,4 | 13,0 | 53,3 |
| 4 | 5 | 2 | 350 | 2 | 6,9 | 6,4 | 92,5 |
| 5 | 4 | 4 | 400 | 2 | 13,6 | 12,2 | 89,6 |
| 6 | 4 | 4 | 450 | 6 | 29,2 | 22,5 | 77,2 |
| 7 | 4 reg. | 4 | 450 | 6 | 49,3 | 30,8 | 62,5 |
| 8 | 5 | 2 | 400 | 2 | 6,9 | 3,2 | 45,5 |
| 9 | 6 | 2 | 400 | 2 | 6,5 | 5,6 | 85,9 |
|  |  |  |  | 4 | 4,3 | 3,7 | 84,3 |
| 10 | 7 | 2 | 350 | 2 | 11,1 | 6,5 | 58,9 |
| V 11 | 8 | 2 | 450 | 2 | 1,7 | 0,4 | 21,8 |
| 12 | 9 | 2 | 450 | 2 | 5,2 | 3,9 | 75,1 |
| V 13 | 10 | 4 | 350 | 2 | 1,0 | 1,0 | 98,4 |
|  |  |  | 450 | 4 | 3,1 | 2,5 | 82,2 |

Die Vergleichsbeispiele 1 und 12 zeigen, daß bei höheren Temperaturen in geringem Umfang auch eine Säurenkatalyse durch die Broensted-Zentren der Zeolithe möglich ist. Diese jedoch erst bei sehr hohen Temperaturen mögliche Katalyse bleibt aber im Umsatz weit hinter der erfindungsgemäßen Basenkatalyse zurück.

**Beispiel V 14 (Vergleichsbeispiel)**

Anstelle von Propargylakohol wurde 2-Butin-1-ol (b.p. 142-143°C) eingesetzt. Es wurde Katalysator 2 bei 400°C entsprechend den Maßgaben des Beispiels 2 verwendet. Die denkbare Isomerisierung zu Crotonaldehyd fand nicht statt. Es fand nur ein Umsatz von 4 % zu wertlosen Leichtsiedern (GC-Analyse) statt. Überraschenderweise ist also die erfindungsgemäße Reaktion nur für Propargylalkohol spezifisch.

**Beispiel 15**

Verwendung von Propargylalkohol als Depotsubstanz für Acrolein. Diels-Alder Reaktion von Propargylalkohol mit Dicyclopentadien ($C_{10}H_{12}$) zum Norbornencarboxaldehyd.

Es wurde unter Verwendung von Katalysator 2 wie im Beispiel 2 verfahren, jedoch wurde nur eine Temperatur von 350°C eingestellt. Mit einer Dosierpumpe wurden 10 ml/h einer Lösung von Propargylalkohol und Dicyclopentadien im Molverhältnis 2 : 1 in den Reaktor eingetragen und verdampft. Der Umsatz an Propargylalkohol betrug 18 %. Im Kondensat wurden neben Acrolein (12 Fl-% im GC) und Resten an Dicyclopentadien (2 Fl-% im GC) 4 Fl-% des gewünschten Norbornenaldehyd gaschromatographisch nachgewiesen. Das Beispiel zeigt, daß Propargylalkohol als Speichersubstanz für Acrolein dienen und entsprechend als in-situ-Quelle für Acrolein verwendet werden kann.

**Herstellung der Katalysatoren**

**Katalysator 1 (Vergleichskatalysator)**

Es wurde ein handelsüblicher acider H-ZSM-5 (Pentasil-Typ) mit einem molaren Verhältnis von $SiO_2/Al_2O_3$ von 57 und einer elektronenmikroskopisch bestimmten Kristallgröße von kleiner als 0,05 μm eingesetzt. Mit diesem Material wurden durch Verformen mit einem Verformungshilfsmittel 2-mm-Stränge hergestellt, die bei 110°C 16 Stunden getrocknet und 16 Stunden bei 500°C calciniert wurden. Im katalytischen Test nach Dessau wurde bei 350°C ein 100%iger Umsatz an Acetonylaceton mit 95 % Selektivität zum 2,3-Dimethylfuran gemessen. Der pH-Wert des Katalysators lag bei 3,1.

**Katalysator 2**

10 g Katalysator 1 wurden mit 9 ml einer 1 normalen KOH-Lösung vermischt und bei 110°C getrocknet. Der pH-Wert lag bei 9,2.

**Katalysator 3**

10 g Katalysator 1 wurden mit 25 ml einer 0,5 normalen NaOH-Lösung vermischt und bei 110°C getrocknet. Der pH-Wert lag bei 10,8.

**Katalysator 4**

3 g Natriummethylat wurden in 50 ml Methanol gelöst und in einem Rotationsverdampfer zu 15 g Katalysator hinzugegeben. Nach dem Einrotieren zur Trockene wurde das Katalysatorpulver 3 Stunden bei 110°C im Trockenschrank getrocknet.

Der pH des Pulvers wurde zu 9,2 bestimmt. Im katalytischen Test nach Dessau wurde bei 350°C ein 100 %iger Umsatz an Acetonylaceton mit 93 % Selektivität zum 3-Methylcyclopentenon gemessen.

**Katalysator 4 reg.**

Nach der Verwendung von Katalysator 4 im Beispiel 6 wurde der Katalysator im Festbettreaktor 12 Stunden im Luftstrom bei 500°C regeneriert und in dieser Form für das Beispiel 7 verwendet (reg = regeneriert).

Katalysator 5

30 g Katalysator 1 wurden bei 80°C unter Rühren in 550 ml Wasser aufgeschlämmt und innerhalb 30 Minuten 6 g CsOH hinzugefügt. Danach wurde 2 Stunden bei 80°C gerührt, filtriert und der Filterkuchen bei 110 bis 120°C getrocknet.

Anschließend wurde das Pulver 4 Stunden bei 500°C calciniert. Der pH-Wert wurde zu 10,4 bestimmt. Im katalytischen Test nach Dessau wurde bei 350°C ein 89 %iger Umsatz an Acetonylaceton mit 94 % Selektivität zum 3-Methylcyclopentenon gemessen.

Katalysator 6

Es wurde handelsübliches MgO verwendet. Mit diesem Material wurden durch Verformen mit einem Verformungshilfsmittel 2-mm-Stränge hergestellt, die bei 110°C 16 Stunden bei 500°C calciniert wurde. Im katalytischen Test nach Dessau wurde bei 350°C ein 99 %iger Umsatz an Acetonylaceton mit 92 % Selektivität zum 3-Methylcyclopentenon gemessen. Der pH-Wert lag bei 11,1.

Katalysator 7 (klein gebrochene Marmorstücke)

Es wurde handelsübliches CaO verwendet. Im katalytischen Test nach Dessau wurde bei 450°C ein 2 %iger Umsatz an Acetonylaceton mit 86 % Selektivität zum 3-Methylcyclopentenon und 14 %iger Selektivität zum 2,5-Dimethylfuran gemessen. Der pH-Wert lag bei 12,8.

Katalysator 8

Handelsübliches $SiO_2$ (D 11-10), welches für Katalysator 9 als Trägermaterial verwendet wurde. Im katalytischen Test nach Dessau wurde bei 350°C ein 25 %iger Umsatz an Acetonylaceton mit 74 % Selektivität zum 3-Methylcyclopentenon und 21 %iger Selektivität zum 2,5-Dimethylfuran gemessen. Der pH-Wert lag bei 7,3.

Katalysator 9

30 g eines handelsüblichen $SiO_2$ (D 11-10), welches auch als Katalysator 8 eingesetzt wurde, wurden im Rotationsverdampfer mit 100 ml einer 0,05 normalen Lösung von $Ba(OH)_2$ (Barytwasser) getränkt und zur trockene einrotiert. Nach dem Trocknen bei 110°C wurde 4 Stunden bei 500°C calciniert.

Im katalytischen Test nach Dessau wurde für den Katalysator 9 bei 350°C ein 77 %iger Umsatz an Acetonylaceton mit 89 % Selektivität zum 3-Methylcyclopentenon und 6 %iger Selektivität zum 2,5-Dimethylfuran gemessen. Der pH-Wert lag bei 9,8.

Vergleichskatalysator 10 (aus ZBM-20/17)

Ein Eisensilicatzeolith vom Pentasil-Typ wird unter hydrothermalen Bedingungen bei autogenem Druck und 165°C aus 273 g Natrium-Wasserglas (27,2 % $SiO_2$, 8,5 % $Na_2O$), 124 g 1,6-Diaminohexan, 521 g Wasser, 20,6 g 96 %iger Schwefelsäure und 16,1 g Eisen(III)-sulfat in einem Rührautoklaven hergestellt. Nach Abfiltrieren und Auswaschen wird das kristalline Reaktionsprodukt bei 110°C/24 h getrocknet und bei 500°C/24 h calciniert. Dieser Eisensilicatzeolith setzt sich aus 88,1 Gew-% $SiO_2$, 7,1 Gew-% $Fe_2O_3$ und 0,074 Gew-% Na zusammen. Mit diesem Material werden durch Verformen mit einem Verformungshilfsmittel 4-mm-Stränge hergestellt, die bei 110°C/16 h getrocknet und bei 500°C/24 h calciniert werden. Die Stränge werden viermal mit 20 gew.-%iger $NH_4Cl$-Lösung (15 ml Lösung/g Formkörper) bei 80°C in einem Steigrohr ausgetauscht. Anschließend wird chloridfrei gewaschen. Die Stränge werden bei 110°C 10 h getrocknet und anschließend bei 500°C 5 h calciniert. Der Na-Gehalt beträgt nun 0,015 Gew.-%. Der pH-Wert lag bei 3,2.

**Patentansprüche**

1. Verfahren zur Isomerierung von Propargylalkohol zu Acrolein, dadurch gekennzeichnet, daß man Propargylalkohol an Heterogenkatalysatoren, die alkalisch reagierende Metallionen enthalten, in der Gasphase bei Temperaturen von 300 bis 550°C und Drücken von 0,01 bis 50 bar umlagert.

2. Verfahren nach Anspruch 1, dadurch gekennzeichnet, daß man Heterogenkatalysatoren verwendet, die Alkalimetallionen der Elemente Li, K, Na, Cs und/oder Erdalkalikationen der Elemente Mg, Ca, Sr, Ba enthalten.

3. Verfahren nach Anspruch 1, dadurch gekennzeichnet, daß man als Heterogenkatalysatoren Molekularsiebe oder strukturverwandte Aluminiumphosphate verwendet, die Alkalimetallionen der Elemente Li, K, Na, Cs und/oder Erdalkalikationen der Elemente Mg, Ca, Sr, Ba enthalten.

4. Verfahren nach Anspruch 1, dadurch gekennzeichnet, daß man als Heterogenkatalysatoren mit Alkali- und/oder Erdalkalionen dotierte Trägermaterialien verwendet.

5. Verfahren nach Anspruch 1, dadurch gekennzeichnet, daß man als Heterogenkatalysator Erdalkalioxide verwendet.

6. Verfahren nach Anspruch 1, dadurch gekennzeichnet, daß man Propargylalkohol als Depotsubstanz zur Freisetzung von Acrolein in chemischen Synthesen verwendet.

7. Verwendung von Propargylalkohol als Depotsubstanz zur Freisetzung von Acrolein in chemischen Synthesen.